# EUROPÄISCHE PATENTSCHRIFT

(11) **EP 2 296 754 B1**
(45) Veröffentlichungstag und Bekanntmachung des Hinweises auf die Patenterteilung: **13.01.2021**
(21) Anmeldenummer: 09768966.5
(22) Anmeldetag: 23.06.2009
(51) Int. Cl.: A61N 2/02, A61N 2/04, A61N 2/06

(54) **VORRICHTUNG ZUR MAGNETFELDTHERAPIE**
APPARATUS FOR MAGNETIC FIELD THERAPY
DISPOSITIF DE THÉRAPIE PAR CHAMPS MAGNÉTIQUES

(30) Priorität: 23.06.2008 DE 102008029415
(43) Veröffentlichungstag der Anmeldung: 23.03.2011
(60) Teilanmeldung: 20206257.6
(73) Patentinhaber: Muntermann, Axel, 35580 Wetzlar-Nauborn (DE)
(72) Erfinder: Muntermann, Axel, 35580 Wetzlar-Nauborn (DE)
(74) Vertreter: Blumbach · Zinngrebe Patentanwälte PartG mbB
(86) Internationale Anmeldenummer: PCT/EP2009/004509
(87) Internationale Veröffentlichungsnummer: WO 2009/156117

(56) Entgegenhaltungen:
- EP-A2- 0 228 537
- WO-A1-2005/075019
- WO-A1-2006/024602
- WO-A2-2008/073512
- DE-A1- 2 610 589
- DE-A1- 19 827 736
- DE-U1- 9 111 936
- JP-A- 2003 260 026

## Beschreibung

### Gebiet der Erfindung

Die Erfindung betrifft eine Vorrichtung zur Magnetfeldtherapie.

### Hintergrund der Erfindung

Verfahren zur Magnetfeldtherapie sowie zugehörige Vorrichtungen zur Durchführung einer solchen sind bekannt.

Ein Verfahren zur Magnetfeldtherapie, bei welchem die therapeutische Wirkung auf dem Effekt der Kernspinresonanz beruht, ist in der europäischen Patentschrift EP 1 089 792 B1 (Patentinhaber Axel Muntermann) beschrieben.

Bei dem derartigen Verfahren wird eine Vorrichtung verwendet, bei welcher ein im Wesentlichen statisches Magnetfeld von einem senkrecht dazu stehenden magnetischen Wechselfeld überlagert wird.

Zur Erzielung einer Kernspinresonanz besteht die Anforderung, dass zumindest das statische Magnetfeld in der Behandlungszone eine möglichst homogene Feldstärke aufweist, da ansonsten eine Kernspinresonanz nur in einem Teilbereich der Behandlungszone stattfindet.

Zur Erzeugung von magnetischen Wechselfeldern wird in der Praxis beispielsweise eine Helmholtzspule verwendet, also eine Anordnung aus zwei Ring- oder Zylinderspulen, die voneinander beabstandet sind.

Nachteilig an einer solchen Spulenanordnung oder an einer Ringspule im Allgemeinen ist, dass sich der Patient während der Behandlung in die Spule hineinlegen muss. Dies ist insbesondere für ältere oder fettleibige Menschen unangenehm, weiter kann die sich über den Patienten erstreckende ringförmige Anordnung Platzangst auslösen.

Auch bei der Behandlung von Tieren ist die Handhabbarkeit einer Ringspule unpraktisch, da das Tier beispielsweise auf einem Schlitten in die Spule hineingefahren werden muss und so in vielen Fällen fixiert werden müsste oder die zur Behandlung notwendige Luftspule über das zu behandelnde Gelenk oder den zu behandelnden Bereich geschoben werden muss.

WO 2009/156117 offenbart eine Vorrichtung mit verschiedenen magnetischen Feldern zur therapeutischen lokalisierten Behandlung, z.B. im Kopfbereich des Patienten. Die Vorrichtung kann flexibel an die Anatomie des Patienten angepasst werden.

### Aufgabe der Erfindung

Der Erfindung liegt demgegenüber die Aufgabe zugrunde, die genannten Nachteile des Standes der Technik zumindest zu reduzieren.

Insbesondere ist es eine Aufgabe der Erfindung, eine Vorrichtung zur Magnetfeldtherapie bereitzustellen, deren Handhabbarkeit verbessert ist. Dabei soll ein möglichst großes Behandlungsvolumen erhalten bleiben.

Eine weitere Aufgabe der Erfindung ist es, eine einfach herzustellende Vorrichtung zur Magnetfeldtherapie bereitzustellen, welche sich aufgrund der guten Homogenität des Feldes zur Durchführung von auf dem Kernspinresonanz-Effekt beruhenden Therapieformen eignet.

Es ist eine weitere Aufgabe der Erfindung, die Zugänglichkeit des Behandlungsbereiches für Patienten zu verbessern.

Eine weitere Aufgabe der Erfindung ist es, den Behandlungsbereich einer Vorrichtung zur Magnetfeldtherapie mit Ausnahme einer Liegefläche möglichst offen und von allen Seiten zugänglich zu halten.

### Zusammenfassung der Erfindung

Die Aufgabe der Erfindung wird bereits durch eine Vorrichtung zur Magnetfeldtherapie nach dem unabhängigen Anspruch gelöst.

Bevorzugte Ausführungsformen und Weiterbildung der Erfindung sind den jeweiligen Unteransprüchen zu entnehmen.

Die Erfindung betrifft eine Vorrichtung zur Magnetfeldtherapie, insbesondere betrifft die Erfindung eine Vorrichtung, welche zur Erzielung von Kernspinresonanzen in dem zu behandelnden Gewebe ausgebildet ist.

Die Vorrichtung weist eine vorzugsweise im Wesentlichen horizontal angeordnete Grundfläche auf, welche insbesondere als Liegefläche für einen Patienten oder das Körperteil eines Patienten ausgebildet ist.

Weiter umfasst die Vorrichtung zwei an der Grundfläche randseitig angeordnete, sich vorzugsweise im Wesentlichen vertikal von den Seiten der Grundfläche aus erstreckende abgewinkelte und/oder bogenförmige Seitenteile.

In den Seitenteilen ist jeweils eine Spule integriert, deren Mittelachse quer, vorzugsweise im Wesentlichen senkrecht zum jeweiligen Seitenteil verläuft.

Die Anordnung der Spulen in den Seitenteilen ermöglicht eine Ausbildung der Vorrichtung, bei welcher die Vorrichtung nach oben offen ist.

Weiter lässt sich durch die Integration von zwei Spulen in den Seitenteilen ein magnetisches Feld mit recht guter Homogenität erzeugen. Da sich das magnetische Feld von einem Seitenteil zum anderen im Wesentlichen gradlinig erstreckt, lässt sich ein recht großes Behandlungsvolumen bereitstellen, in welchem Kernspinresonanzen erzielt werden. Die Spulen sind also als eine Art Helmholtz-Spulenpaar ausgebildet.

Die gesamte Vorrichtung ist vorzugsweise in etwa U-förmig ausgebildet.

Gegenüber bekannten Vorrichtungen zur Magnetfeldtherapie mit Ring- oder Zylinderspulen ermöglicht die Erfindung eine Zugänglichkeit des Behandlungsbereiches von oben. So muss der Patient nicht in die Spulenanordnung hinein geschoben werden, sondern kann sich aus eigener Kraft zurücklegen und so mit zumindest einem Körperteil in die Behandlungszone eindringen.

Auch bei der Behandlung von Tieren ermöglicht die vereinfachte Zugänglichkeit eine wesentlich leichtere Behandlung. So kann z. B. ein Hund oder ein Kleintier von oben in den Behandlungsbereich eingebracht werden und während der Behandlung zusätzlich betreut werden und muss nicht auf einer Liege festgeschnallt oder anderweitig fixiert werden.

Weiter umfasst die Vorrichtung eine erste Einrichtung zum Erzeugen eines magnetischen Feldes, wobei das von dieser ersten Einrichtung erzeugte magnetische Feld quer, vorzugsweise im Wesentlichen senkrecht zu dem von den Spulen erzeugten Feld steht.

Durch Kombination eines im Wesentlichen statischen Feldes mit einem überlagernden Wechselfeld können so Kernspinresonanzen im zu behandelnden Gewebe erzeugt werden.

Bei einer bevorzugten Ausführungsform der Erfindung umfasst die erste Einrichtung zum Erzeugen eines magnetischen Feldes zumindest eine in oder unter der Grundfläche angeordnete Spule mit im Wesentlichen flachem Querschnitt.

Bei dieser Ausführungsform wird das von der ersten Einrichtung zum Erzeugen eines magnetischen Feldes erzeugte magnetische Feld nicht durch einen Permanentmagneten, sondern ebenfalls mittels einer Spule erzeugt.

Vorzugsweise wird hierzu eine in der Grundfläche flach gelegte im Wesentlichen flache Zylinder- oder Ringspule verwendet.

Es versteht sich, dass Teile eines Spulensystems aber auch noch bis in die Seitenteile hineinragen können.

Bei einer Ausführungsform der Erfindung wird durch die flach gelegte Spule das im Wesentlichen statische Magnetfeld erzeugt, wohingegen über die in den Seitenteilen integrierten Spulen das im Wesentlichen dazu stehende magnetische Wechselfeld erzeugt wird. Bei einer alternativen Ausführungsform, bei welcher das Wechselfeld über die erste Einrichtung zur Erzeugung eines magnetischen Feldes generiert wird, kommt der Vorrichtung die gute Homogenität des Feldes zwischen den Seitenteilen zugute.

Auch eine Kombination von Permanentmagneten und Spulen zur Erzeugung des im Wesentlichen statischen Magnetfeldes ist im Sinne der Erfindung denkbar.

Die Vorrichtung zur Magnetfeldtherapie ist vorzugsweise gegenüber der Grundfläche, also insbesondere nach oben, geöffnet, um einen leichteren Zugang zu ermöglichen. Die beschriebenen Spulensysteme können in ein geschlossenes Gehäuse mit oder ohne Durchbrüche eingebaut sein.

Bei einer weiteren bevorzugten Ausführungsform weisen die Seitenteile jeweils eine Aussparung auf, wobei die Spule jeweils um die Aussparung herum läuft.

Diese Ausführungsform ermöglicht eine Ausgestaltung, bei welcher die Seitenteile im Wesentlichen lediglich einen Rahmen zum Aufnehmen der Spule bilden.

Die Aussparung hat zum einen den Zweck, dass die Behandlungszone von dem Patienten nicht wie ein enger Tunnel wahrgenommen wird, wie dies bei bekannten ringförmigen Ausgestaltungen der Fall ist.

Zum anderen kann durch die Aussparungen Licht eindringen, so dass es auch in der Behandlungszone hell ist.

Bei einer Weiterbildung der Erfindung ist eine Beleuchtungsvorrichtung vorgesehen, über welche die Behandlungszone durch ein Lichtfeld markiert wird.

Über eine vorzugsweise außen angebrachte Schalter-/Tasteranordnung können bei einer bevorzugten Ausführungsform der Erfindung Lichtquellen für die ordnungsgemäße Lagerung eines Patienten als Positionierhilfe zugeschaltet werden. Weiter ist denkbar auch eine therapiebegleitende Beleuchtung in der Behandlungszone einschalten zu können. Über ein weiteres Leuchtmittel, welches beispielsweise mit zumindest einer weiteren separaten Spule verbunden ist, welche über die Felder der Einrichtung zur Erzeugung eines magnetischen Feldes induktiv versorgt wird, kann die Funktionsfähigkeit der Vorrichtung zur Magnetfeldtherapie angezeigt werden.

Die Aussparungen sind bei einer bevorzugten Ausführungsform im Wesentlichen rechteckig ausgebildet. Auch die Seitenteile besitzen in der Seitenansicht eine im Wesentlichen rechteckige Form. Eine derartige Ausgestaltung ermöglicht es, eine im Wesentlichen rechteckige Behandlungszone mit guter Raumausnutzung bereitzustellen. Die Aussparungen nehmen bei einer bevorzugten Ausführungsform der Erfindung mehr als 50%, vorzugsweise mehr als 60% der Fläche der Seitenteile ein.

Gemäß der Erfindung sind die Seitenteile in der Spulenebene gekrümmt.

Die sich durch den Rahmen der Seitenteile erstreckende Spule, welche als Zylinder- oder Ringspule ausgebildet ist, ist somit mit dem Seitenteil gebogen, was eine bessere Raumausnutzung ermöglicht.

Insbesondere ist eine in der Vorderansicht annähernd kreisförmige Ausgestaltung der Seitenteile vorgesehen.

Für die verschiedenen Behandlungsaufgaben werden vorzugsweise Spulen mit einem Durchmesser zwischen 10 cm und 1 m, besonders bevorzugt zwischen 50 cm und 90 cm verwendet, wobei die Grundfläche der Vorrichtung zur Magnetfeldtherapie eine Länge zwischen 20 und 120 cm, bevorzugt zwischen 40 und 80 cm aufweist.

Die Spulen in den Seitenteilen können sowohl zur Erzeugung eines im Wesentlichen statischen Feldes als auch zur Erzeugung eines magnetischen Wechselfeldes verwendet werden.

Im Falle der Verwendung für ein statisches Feld ist besonders die gute Homogenität des Feldes vorteilhaft, da die erforderliche Frequenz des Wechselfeldes zur Erzielung einer Kernspinresonanz von der Stärke des statischen Feldes abhängt.

Aber auch mit einer in die Grundfläche flach gelegten Spule lässt sich ein Feld mit hinreichender Homogenität erzeugen. Insbesondere kann das statische Feld gesweept werden, also ein Feld mit einer hinreichenden Mindestfeldstärke erzeugt werden, welche variiert wird, so dass durch das Verändern des statischen Feldes sichergestellt ist, dass in jedem Bereich der Behandlungszone während des Sweepens zumindest kurzzeitig die Resonanzbedingung hergestellt wird.

Gleiches kann auch durch ein Sweepen der Frequenz des magnetischen Wechselfeldes erreicht werden.

Grundfläche und Seitenteile mit jeweiligen Spulen sind vorzugsweise als einteiliges Modul, insbesondere aus einem Kunststoff, ausgebildet. Beispielsweise können die Spulen auf besonders einfache Weise in einem Gehäuse aus Spritzguss integriert werden oder insbesondere in einer Spritzgussform mit Kunststoff, Kunststoffschaum oder einem anderen dielektrischen Material ummantelt werden.

Bei einer Weiterbildung der Erfindung umfasst die Vorrichtung zur Magnetfeldtherapie zusätzlich einen auf der Grundfläche angeordneten Schlitten mit einer Liegefläche, welcher relativ zur Grundfläche verschiebbar ist. So ist es möglich, durch Verschieben des Schlittens die Behandlungszone relativ zum Patienten zu verschieben und mehrere Abschnitte des Körpers zu behandeln.

Bei einer alternativen Ausführungsform der Erfindung ist ein aus Grundfläche und Seitenteilen gebildetes Modul bezüglich der Höhe einer Sitzfläche verschiebbar ausgebildet. Bei dieser Ausführungsform der Erfindung ist die Grundfläche im Wesentlichen vertikal angeordnet und lässt sich entlang des Patientenkörpers, der auf der Sitzfläche sitzt, nach oben und unten verschieben. Diese Ausführungsform der Erfindung ist besonders geeignet, um Behandlungen im Kopf oder oberen Rumpfbereich durchzuführen. Besonders zur Hautbehandlung im Gesichtsbereich eignet sich diese Ausführungsform.

Bei einer Weiterbildung der Erfindung ist das Modul schwenkbar ausgebildet und kann so aus der exakt vertikalen Position verkippt werden, um je nach Sitzposition und zu behandelndem Körperbereich optimal eingestellt werden zu können.

### Kurzbeschreibung der Zeichnungen

Die Erfindung soll im Folgenden anhand schematisch dargestellter Ausführungsbeispiele, bezugnehmend auf die Zeichnungen Fig. 1 bis Fig. 5 näher erläutert werden.
- Fig. 1: zeigt schematisch eine perspektivische Ansicht eines Ausführungsbeispiels der Erfindung,
- Fig. 2: zeigt das in Fig. 1 dargestellte Ausführungsbeispiel, wobei schematisch die Anordnung der Spulen dargestellt ist,
- Fig. 3: zeigt schematisch die Anordnung der Spulen,
- Fig. 4: zeigt ein weiteres Ausführungsbeispiel einer Vorrichtung zur Magnetfeldtherapie mit einer verstellbaren Liege.
- Fig. 5: zeigt ein weiteres Ausführungsbeispiel der Erfindung
- Fig. 6: zeigt ein weiteres Ausführungsbeispiel der Erfindung, bei welchem eine Sitzfläche vorgesehen ist.

### Detaillierte Beschreibung der Zeichnungen

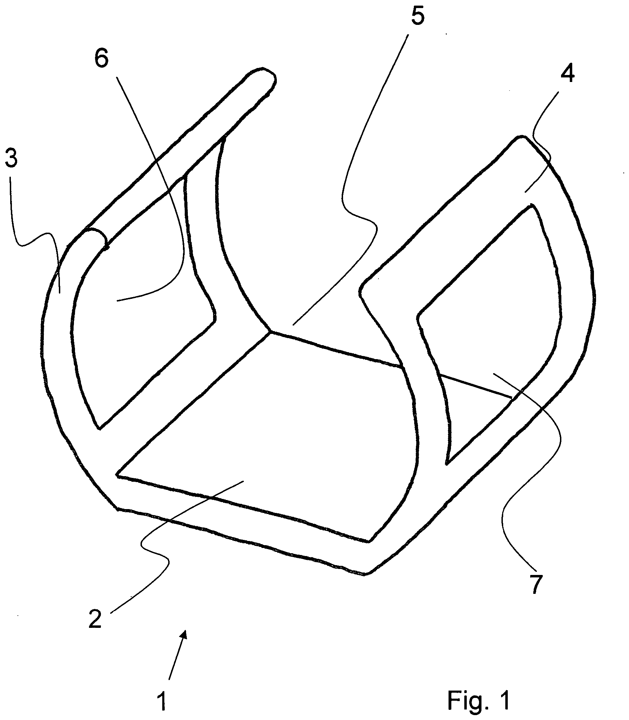

In Fig. 1 ist schematisch eine Vorrichtung zur Magnetfeldtherapie 1 dargestellt.

Die Vorrichtung zur Magnetfeldtherapie 1 umfasst eine Grundfläche 2, welche beispielsweise dazu dient, ein zu behandelndes Körperteil darauf abzulegen.

Von der Grundfläche 2 aus erstrecken sich die beiden Seitenteile 3 und 4.

Die Seitenteile 3 und 4 sind in der Vorderansicht gebogen ausgebildet und haben einen in etwa kreissegmentförmigen beziehungsweise U-förmigen Querschnitt.

Weiter bilden die beiden Seitenteile 3 und 4 einen Rahmen und haben somit jeweils eine Aussparung 6, 7.

Gegenüber der Grundfläche 2 ist die Vorrichtung zur Magnetfeldtherapie 1 offen, was gegenüber bekannten Vorrichtungen zur Magnetfeldtherapie eine wesentlich leichtere Zugänglichkeit der zwischen Seitenteilen 3, 4 und der Grundfläche 2 liegenden Behandlungszone 5 ermöglicht.

In der Grundfläche 2 befindet sich eine erste Einrichtung zur Erzeugung eines magnetischen Feldes (nicht dargestellt).

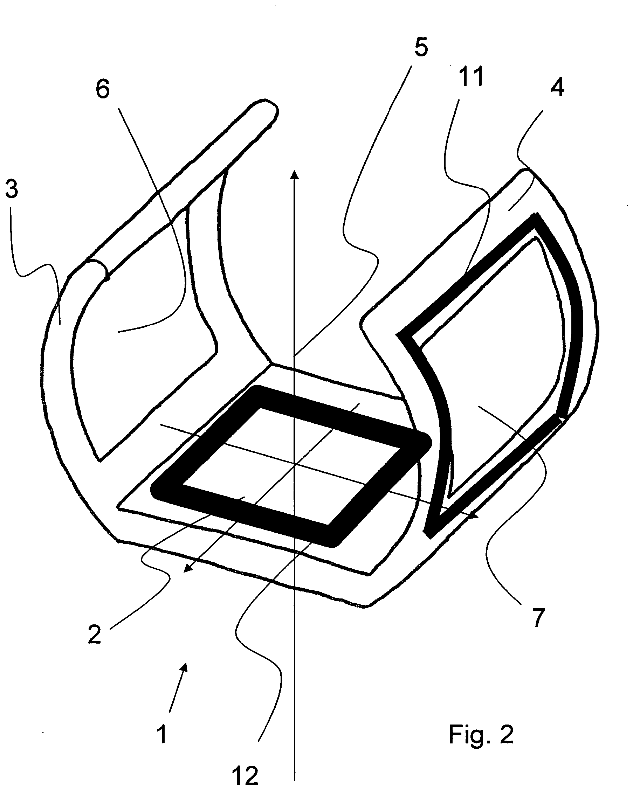

Fig. 2 zeigt die Ausführungsform einer Vorrichtung zur Magnetfeldtherapie 1 gemäß Fig. 1, wobei in dieser Ausführungsform schematisch eine in der Grundfläche 2 integrierte Spule 12 eingezeichnet ist, welche eine erste Einrichtung zur Erzeugung eines magnetischen Feldes bildet.

In dem rechten Seitenteil 4 befindet sich ebenfalls eine Spule 11, über die ein Magnetfeld erzeugbar ist.

Die Spule im Seitenteil 3 ist nicht dargestellt, es ist aber ersichtlich, dass sich über die Spulen im Seitenteil 3 und im Seitenteil 4 ein Magnetfeld erzeugen lässt, welches im Wesentlichen senkrecht zu dem von der Spule 12 erzeugten Magnetfeld steht.

Die Spulen 11, 12 können beispielsweise über ein netzunabhängiges Steuergerät, insbesondere mit einem Akkumulator versorgt werden (nicht dargestellt).

So können innerhalb der Behandlungszone 5 Kernspinresonanzen im zu behandelnden Gewebe (nicht dargestellt) erzeugt werden.

Über die Aussparungen 6 und 7 kann die Behandlungszone 5 über eine Beleuchtung (nicht dargestellt) markiert werden.

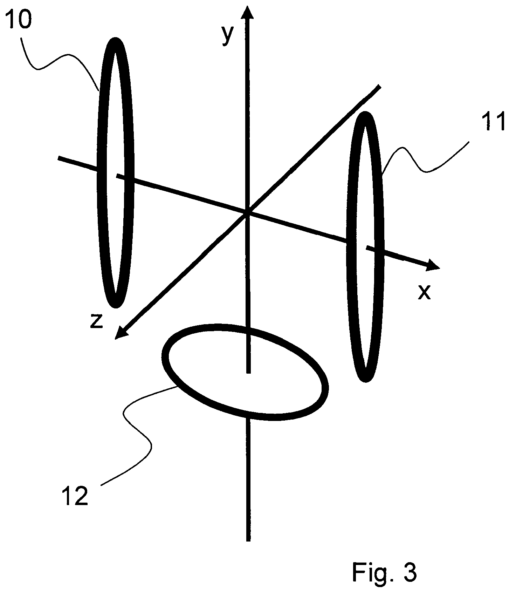

In Fig. 3 ist schematisch die Spulenanordnung dargestellt.

Über die in der Grundfläche integrierte Spule 12 wird ein erstes Magnetfeld erzeugt, dessen Feldlinien in der Behandlungszone im Wesentlichen entlang der Y-Achse verlaufen.

Über die Spulen 10 und 11 wird ein dazu senkrechtes Feld erzeugt, dessen Feldlinien im Wesentlichen entlang der X-Achse verlaufen. Die Spulen 10 und 11 bilden also eine zweite Einrichtung zur Erzeugung eines magnetischen Feldes.

Das von den Spulen 10 und 11 erzeugte Feld ist aufgrund der Anordnung, die einer Helmholtzspule nahekommt, sehr homogen. Es bietet sich daher an, zur Erzeugung von Kernspinresonanzen im zu behandelnden Gewebe über die Spulen 10 und 11 ein im Wesentlichen statisches Magnetfeld zu erzeugen und über die Spule 12 ein senkrecht dazu stehendes Wechselfeld zu erzeugen, welches zum Umklappen der Spins führt.

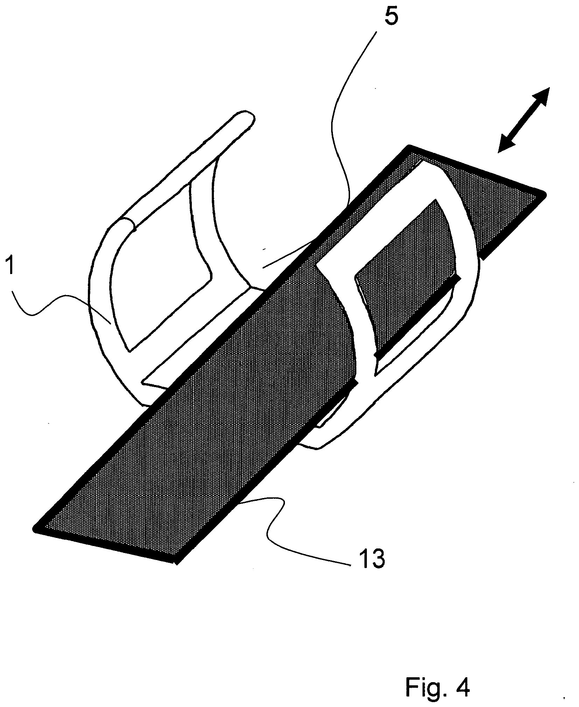

In Fig. 4 ist schematisch eine weitere Ausführungsform der Erfindung dargestellt. Bei dieser Ausführungsform ist die in den Figuren Fig. 1 und Fig. 2 dargestellte Vorrichtung zur Magnetfeldtherapie 1 mit einer entlang der Grundfläche der Vorrichtung beweglichen Liege versehen. Durch eine Bewegung der Liege 13 relativ zur Vorrichtung der Magnetfeldtherapie 1 kann, sofern ein Patient auf der Liege liegt (nicht dargestellt), die Behandlungszone 5 leicht verschoben werden und es können so nacheinander verschiedene Zonen des Körpers behandelt werden.

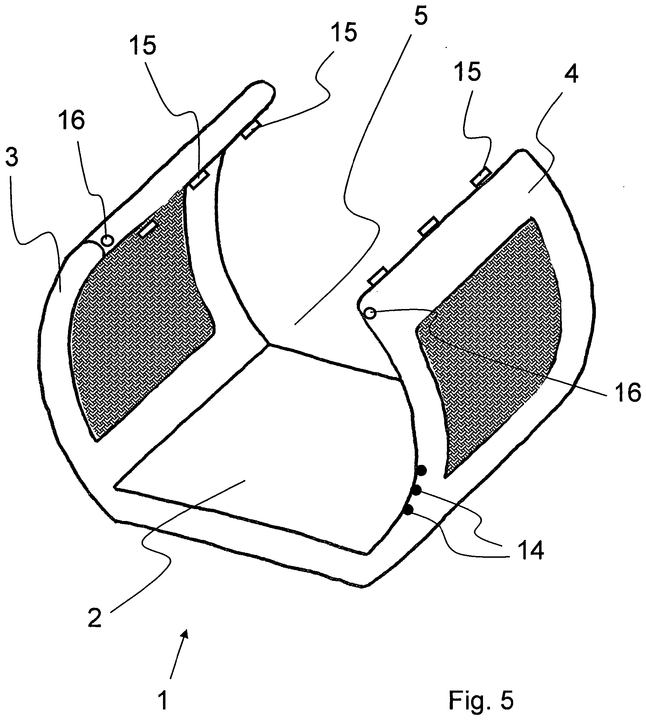

Bezugnehmend auf Fig. 5 soll ein weiteres Ausführungsbespiel der Erfindung näher erläutert werden.

Dargestellt ist ebenfalls eine Vorrichtung zur Magnetfeldtherapie 1, wobei in diesem Ausführungsbeispiel die Seitenteile 3, 4 keine Aussparungen aufweisen, sondern geschlossen sind. Ansonsten entspricht die Grundform der Vorrichtung zur Magnetfeldtherapie 1 der in Fig. 1 dargestellten Vorrichtung.

Zusätzlich ist in dieser Zeichnung eine Anordnung von Schaltern 14 dargestellt, mit der beispielsweise das Gerät bedient werden kann oder Leuchten 15 ein- und ausgeschaltet werden können. Die Leuchten 15 sind vorzugsweise als LED Leuchten ausgebildet. In diesem Ausführungsbeispiel sind randseitig jeweils drei Leuchten vorgesehen, um die Behandlungszone 5 zu markieren.

Weiter verfügt die Vorrichtung zur Magnetfeldtherapie 1 über Funktionsanzeigen 16 anhand derer erkennbar ist, ob das Gerät arbeitet. Die Funktionsanzeigen 16 können beispielsweise eine mit einer weiteren Spule verbundene LED umfassen (nicht dargestellt), wobei die LED bei korrekter Funktion der Vorrichtung über die Spule induktiv versorgt wird.

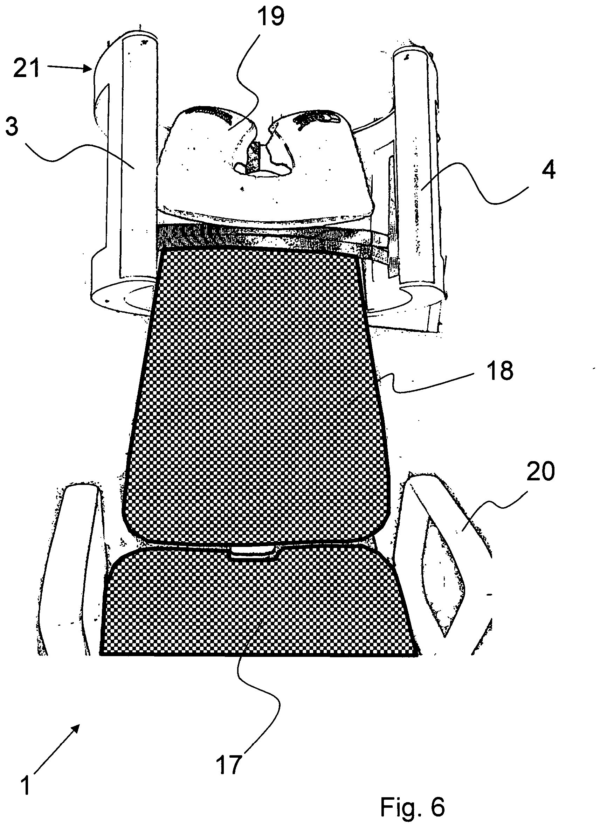

Fig. 6 zeigt eine alternative Ausführungsform der Erfindung, bei welcher die Vorrichtung zur Magnetfeldtherapie 1 eine Sitzfläche 17 aufweist, auf welche sich der Patient setzen kann. Zur Steigerung der Bequemlichkeit umfasst die Vorrichtung noch eine Rückenlehne 18, eine Armlehne 20 sowie ein Nackenkissen 19.

Ein Modul 21, welches Grundfläche und Seitenteile 3, 4 umfasst ist im Wesentlichen vertikal angeordnet. Vorzugsweise ist das Modul 21 zumindest in der Höhe verstellbar, um die Vorrichtung zur Magnetfeldtherapie 1 auf unterschiedliche Patientengrößen anpassen zu können oder um unterschiedliche Körperpartien zu behandeln.

### Bezugszeichenliste

- 1: Vorrichtung zur Magnetfeldtherapie
- 2: Grundfläche
- 3: Seitenteil
- 4: Seitenteil
- 5: Behandlungszone
- 6: Aussparung
- 7: Aussparung
- 10: Spule
- 11: Spule
- 12: Spule
- 13: Liege
- 14: Schalter
- 15: Leuchte
- 16: Funktionsanzeige
- 17: Sitzfläche
- 18: Lehne
- 19: Kissen
- 20: Armlehne
- 21: Modul

## Patentansprüche

1. Vorrichtung zur Magnetfeldtherapie (1), umfassend eine horizontal angeordnete Grundfläche (2) mit einer ersten Einrichtung zum Erzeugen eines magnetischen Feldes, welche eine Spule (12) umfasst, deren Mittelachse im Wesentlichen senkrecht zur Grundfläche (2) verläuft, mit welcher ein magnetisches Wechselfeld erzeugbar ist, wobei die Grundfläche (2) als Liegefläche für einen Patienten oder das Körperteil eines Patienten ausgebildet ist,
und zwei sich vertikal von den Seiten der Grundfläche (2) aus erstreckende abgewinkelte Seitenteile (3,4), wobei die Vorrichtung zur Magnetfeldtherapie gegenüber der Grundfläche (2) offen ist,
wobei die Seitenteile (3,4) jeweils eine sich durch den Rahmen der Seitenteile erstreckende, gebogene Spule (10,11) aufweisen, deren Mittelachse quer zum jeweiligen Seitenteil (3,4) verläuft und
wobei ein von den Spulen (10,11) erzeugtes magnetisches Feld zumindest abschnittsweise senkrecht zu einem von der ersten Einrichtung zum Erzeugen eines magnetischen Feldes erzeugten magnetischen Feldes steht,
und wobei die Seitenteile (3,4) gekrümmt sind,
weiter wobei die Vorrichtung zur Magnetfeldtherapie (1) einen auf der Grundfläche (2) angeordneten Schlitten mit der Liegefläche umfasst, wobei der Schlitten relativ zur Grundfläche (2) verschiebbar ausgebildet ist.

2. Vorrichtung zur Magnetfeldtherapie (1) nach dem vorstehenden Anspruch, **dadurch gekennzeichnet, dass** die Mittelachse der Spulen (3,4) senkrecht zum jeweiligen Seitenteil verläuft.

3. Vorrichtung zur Magnetfeldtherapie (1) nach einem der vorstehenden Ansprüche, **dadurch gekennzeichnet, dass** die erste Einrichtung zum Erzeugen eines magnetischen Feldes zumindest eine in oder unter der Grundfläche angeordnete Spule (12) mit im Wesentlichem flachem Querschnitt umfasst.

4. Vorrichtung zur Magnetfeldtherapie (1) nach einem der vorstehenden Ansprüche, **dadurch gekennzeichnet, dass** die Seitenteile (3,4) jeweils eine Aussparung (6,7) aufweisen und/oder dass die Seitenteile (3,4) rahmenförmig ausgebildet sind.

5. Vorrichtung zur Magnetfeldtherapie (1) nach einem der vorstehenden Ansprüche, **dadurch gekennzeichnet, dass** die Spulen (3,4) einen Durchmesser zwischen 10 cm und 1 m, vorzugsweise zwischen 50 cm und 90 cm aufweisen und/oder dass die Grundfläche (2) eine Länge zwischen 20 und 120 cm, vorzugsweise zwischen 40 und 80 cm aufweist.

6. Vorrichtung zur Magnetfeldtherapie (1) nach einem der vorstehenden Ansprüche, **dadurch gekennzeichnet, dass** über die Spulen (3,4) ein im Wesentlichen statisches Feld erzeugbar ist.

7. Vorrichtung zur Magnetfeldtherapie nach einem der vorstehenden Ansprüche, **dadurch gekennzeichnet, dass** die Grundfläche (2) mit der ersten Einrichtung zum Erzeugen eines magnetischen Feldes und die Seitenteile (3,4) mit den Spulen (10,11) als einteiliges Modul (21) ausgebildet sind.

8. Vorrichtung zur Magnetfeldtherapie (1) nach einem der vorstehenden Ansprüche, **dadurch gekennzeichnet, dass** die Vorrichtung zur Erzeugung von Kernspinresonanzen im zu behandelnden Gewebe ausgebildet ist.

## Claims

1. An apparatus for magnetic field therapy (1), comprising:
a base (2) arranged in a horizontal orientation, with first means for generating a magnetic field, comprising a coil (12) having a central axis that extends substantially perpendicular to said base (2), which is operable to generate a magnetic field, said base (2) being in the form of a support surface for a patient or for a body part of a patient;
and two angled side parts (3, 4) extending vertically from the lateral sides of the base (2), the apparatus for magnetic field therapy being open opposite the base (2);
wherein each of the side parts (3, 4) includes a curved coil (10, 11) extending within the frame of the side parts and having a central axis that extends perpendicular to the respective side part (3, 4); and
wherein a magnetic field produced by said coils (10, 11) is perpendicular to a magnetic field produced by said first means for generating a magnetic field, at least in sections thereof;
and wherein the side parts (3, 4) have a curved shape;
wherein further the apparatus for magnetic field therapy (1) comprises a carriage arranged on the base (2) and having a couch surface, wherein said carriage is adapted to be moveable relative to the base (2).

2. The apparatus for magnetic field therapy (1) according to the preceding claim, **characterized in that** the central axis of the coils (3, 4) extends perpendicular to the respective side part.

3. The apparatus for magnetic field therapy (1) according to any one of the preceding claims, **characterized in that** the first means for generating a magnetic field comprises at least one coil (12) that has a substantially flat cross section and is arranged within or below the base.

4. The apparatus for magnetic field therapy (1) according to any one of the preceding claims, **characterized in that** the side parts (3, 4) each have a recess (6, 7); and/or that the side parts (3, 4) have a frame-like shape.

5. The apparatus for magnetic field therapy (1) according to any one of the preceding claims, **characterized in that** the coils (3, 4) have a diameter between 10 cm and 1 m, preferably between 50 cm and 90 cm; and/or that the base (2) has a length between 20 and 120 cm, preferably between 40 and 80 cm.

6. The apparatus for magnetic field therapy (1) according to any one of the preceding claims, **characterized in that** the coils (3, 4) are operable to generate a substantially static field.

7. The apparatus for magnetic field therapy (1) according to any one of the preceding claims, **characterized in that** the base (2) together with the first means for generating a magnetic field and the side parts (3, 4) with the coils (10, 11) are designed to form a unitary module (21).

8. The apparatus for magnetic field therapy (1) according to any one of the preceding claims, **characterized in that** the apparatus is operable to generate nuclear magnetic resonances in the tissue to be treated.

## Revendications

1. Dispositif de thérapie par champ magnétique (1), comprenant
une surface de base (2) disposée horizontalement, avec un premier dispositif destiné à générer un champ magnétique et comportant une bobine (12) dont l'axe médian s'étend de façon sensiblement perpendiculaire à la surface de base (2) et qui permet de générer un champ magnétique alternatif, la surface de base (2) étant réalisée sous forme de surface d'appui pour un patient ou la partie du corps d'un patient,
et deux éléments latéraux (3, 4) coudés qui s'étendent verticalement à partir des côtés de la surface de base (2), le dispositif de thérapie par champ magnétique étant ouvert par rapport à la surface de base (2),
les éléments latéraux (3, 4) présentant chacun une bobine (10, 11) incurvée qui s'étend dans le cadre des éléments latéraux et dont l'axe médian s'étend transversalement à l'élément latéral (3, 4) respectif, et
un champ magnétique généré par les bobines (10, 11) étant perpendiculaire, au moins par portions, à un champ magnétique généré par le premier dispositif destiné à générer un champ magnétique,
et les éléments latéraux (3, 4) étant incurvés,
le dispositif de thérapie par champ magnétique (1) comportant en outre un chariot disposé sur la surface de base (2) et comprenant la surface d'appui, le chariot étant réalisé de manière à pouvoir être déplacé par rapport à la surface de base (2).

2. Dispositif de thérapie par champ magnétique (1) selon la revendication précédente, **caractérisé en ce que** l'axe médian des bobines (3, 4) s'étend perpendiculairement à l'élément latéral respectif.

3. Dispositif de thérapie par champ magnétique (1) selon l'une des revendications précédentes, **caractérisé en ce que** le premier dispositif de génération d'un champ magnétique comprend au moins une bobine (12) qui est disposée dans ou sous la surface de base (2) et présente une section transversale sensiblement plate.

4. Dispositif de thérapie par champ magnétique (1) selon l'une des revendications précédentes, **caractérisé en ce que** les éléments latéraux (3, 4) présentent chacun un évidement (6, 7) et/ou **en ce que** les éléments latéraux (3, 4) sont réalisés en forme de cadres.

5. Dispositif de thérapie par champ magnétique (1) selon l'une des revendications précédentes, **caractérisé en ce que** les bobines (3, 4) présentent un diamètre compris entre 10 cm et 1 m, de préférence entre 50 cm et 90 cm, et/ou **en ce que** la surface de base (2) présente une longueur comprise entre 20 et 120 cm, de préférence entre 40 et 80 cm.

6. Dispositif de thérapie par champ magnétique (1) selon l'une des revendications précédentes, **caractérisé en ce que** les bobines (3, 4) permettent de générer un champ sensiblement statique.

7. Dispositif de thérapie par champ magnétique selon l'une des revendications précédentes, **caractérisé en ce que** la surface de base (2), avec le premier dispositif de génération d'un champ magnétique, et les éléments latéraux (3, 4), avec les bobines (10, 11), sont réalisés sous forme de module monobloc (21).

8. Dispositif de thérapie par champ magnétique (1) selon l'une des revendications précédentes, **caractérisé en ce que** le dispositif est conçu pour la génération de résonance magnétique nucléaire dans le tissu à traiter.
